# EUROPÄISCHE PATENTANMELDUNG

(11) **EP 3 360 590 A1**
(43) Veröffentlichungstag der Anmeldung: **15.08.2018**
(21) Anmeldenummer: 18156374.3
(22) Anmeldetag: 12.02.2018
(51) Int. Cl.: A61M 5/20

(54) **KIT-OF-PARTS**

(30) Priorität: 13.02.2017 DE 102017102765
(71) Anmelder: Götz, Rudolf, 85416 Langenbach/Oberhummel (DE)
(72) Erfinder: Götz, Rudolf, 85416 Langenbach/Oberhummel (DE)
(74) Vertreter: MayReiprich Patentanwälte

(57) **Zusammenfassung**

Gegenstand der Erfindung ist ein Kit-of-Parts, umfassend eine Vorrichtung für nadellose Injektionen in schneller Abfolge mit einer Einrichtung zum Identifizieren des mittels der Vorrichtung zu verabreichenden Wirkstoffs sowie einer Auslöseeinrichtung zum Auslösen einer Injektion und ein Behältnis mit einem Lokalanästhetikum, das mit der Vorrichtung verbindbar ist und eine Einrichtung zum Identifizieren des Lokalanästhetikums und der Charge desselben aufweist.

## Beschreibung

Die vorliegende Erfindung betrifft einen Kit-of-Parts, umfassend eine Vorrichtung für nadellose Injektionen in schneller Abfolge und ein Behältnis mit einem Lokalanästhetikum.

Im Stand der Technik sind verschiedene Vorrichtungen für nadellose Injektionen in schneller Abfolge bekannt und werden üblicherweise beim Impfen von Nutztieren eingesetzt, insbesondere beim Impfen größerer Nutztierbestände, also beispielsweise in der Schweine- oder Rindermast.

Lokalanästhetika wurden mittels einer derartigen Vorrichtung bisher nicht verabreicht.

Es wurde nun überraschend gefunden, dass das Bereitstellen des oben beschriebenen Kit-of-Parts insbesondere für das Kastrieren von Jungebern erhebliche Vorteile für das Tierwohl und auch wirtschaftlicher Art bietet.

Seit geraumer Zeit werden Alternativen zur betäubungslosen Kastration von unter 8 Tage alten männlichen Ferkeln diskutiert, da die betäubungslose Kastration nicht mit dem modernen Tierwohlgedanken vereinbar ist.

In Deutschland werden in diesem Zusammenhang hauptsächlich vier Alternativen diskutiert. Dies sind die Ebermast, eine Impfung gegen Ebergeruch, eine intramuskuläre Vollnarkose sowie eine Inhalationsnarkose.

Die Ebermast setzt eine getrennt geschlechtliche Aufstallung, Vermarktung und Transport von männlichen und weiblichen Schweinen voraus, da die Mast ausschliesslich mit männlichen Schweinen erfolgt.

Als Folge ergeben sich hieraus einige erhebliche Nachteile, wie beispielsweise eine generelle Kostensteigerung durch den erforderlichen logistischen Mehraufwand sowie die erforderliche Geruchsdetektion nach der Schlachtung. Ferner existiert eine besondere Tierschutzproblematik aufgrund des natürlichen Sexualverhaltens der Eber, die sich im sogenannten Aufspringen und im Penisbeissen zeigt, da weder chirurgisch, noch medikamentös in die Hormonentwicklung der Tiere eingegriffen wird.

Weiterhin ist die Qualität des aus der Ebermast resultierenden Fleischs allgemein verschlechtert, weil das Fleisch einen höheren Wassergehalt aufweist und das Fett durch einen größeren Anteil an ungesättigten Fettsäuren weicher und schmieriger wird. Beides ist für sowohl für die weitere kommerzielle Verarbeitung, als auch für den Verbraucher nicht akzeptabel.

Ausserdem ist nachteilig, dass kleine Betriebe aufgrund des zusätzlichen Aufwands eine Ebermast nur schwer wirtschaftlich durchführen können und unter Umständen aus dem Markt gedrängt werden.

Bei der Impfung gegen Ebergeruch erfolgt zwar keine Hormonbehandlung, doch die Impfung wird insbesondere vom Verbraucher schlecht akzeptiert, da in die hormonelle Geschlechtsentwicklung der Tiere eingegriffen wird.

Praktisch wird ein synthetisches und modifiziertes Analogon des Hormons Gonadoliberin verwendet, das aufgrund der Modifikation eine Immunantwort beim Schwein auslöst, die sich auch gegen das körpereigene Gonadoliberin richtet und so letztendlich die Testosteronbildung unterbindet.

Abgesehen von der mangelnden Akzeptanz beim Verbraucher, ist ferner das Erfordernis einer wenigstens zweimaligen Injektion nachteilig. Dies ist wegen des zusätzlichen personellen Aufwands sowie der durch den Impfstoff verursachten Kosten zumindest wirtschaftlich belastend für den Mastbetrieb, wobei weiterhin eine Geruchskontrolle am Schlachtband erforderlich bleibt.

Ausserdem ist derzeit nur ein Impfstoff für die Impfung gegen Ebergeruch kommerziell erhältlich. Dieser wird unter der Marke Improvac® von der Firma Zoetis vertrieben.

Schliesslich liegt die Erfolgsquote bei dieser Impfung nur zwischen 92 und 97 %, da aus Arbeitsschutzgründen mit einem Sicherheitsinjektor gearbeitet wird um eine versehentliche Selbstimpfung zu vermeiden.

Die Vollnarkose per i.m. Injektion mit Ketamin und Azaperon ist bereits deshalb recht kostenintensiv, da sie nur von einem Tierarzt durchgeführt werden darf. Sie ist ferner aufgrund der langen Nachschlafphase und der daraus resultierenden Gefahr der Unterkühlung und der Gefahr des Erdrückens durch die Sau problematisch. Hinzu kommen ein erheblicher Aufwand für die erforderlichen, zusätzlichen Boxen.

Die Inhalationsnarkose mit Isofluran darf ebenfalls nur vom Tierarzt durchgeführt werden, verursacht hohe Gerätekosten und bedingt einen hohen Zeitaufwand. Weiterhin ist Isofluran Iebertoxisch, was für den Anwender oder Betriebsangehörige von Bedeutung ist, da der Wirkstoff durch die Tiere abgeatmet wird.

Schliesslich ist nicht unerheblich, dass nur etwas 70 % der behandelten Tiere eine ausreichende oder erwünschte Narkosetiefe erreichen, so dass insgesamt nur eine unzureichende Schmerzausschaltung erfolgt, wobei die Narkosetiefe trotzdem zusätzlich überwacht werden muss.

Eine in Deutschland nicht durchgeführte Methode ist das Kastrieren unter Lokalanästhesie, bei der eine, an sich schon sehr schmerzhafte, intratestikuläre Injektion eines Lokalanästhetikums in beide Hoden erfolgt. Diese Methode wird in Skandinavien praktiziert, insbesondere in Norwegen.

Neben den bereits erwähnten Schmerzen durch die Injektion ist auch nachteilig, dass relativ große Injektionsvolumina von ca. 0,5 ml pro Injektion erforderlich sind und eine genaue Applikation hinsichtlich Injektionstiefe und Injektionsrichtung wichtig ist.

Des weiteren besteht die Gefahr von üblichen, mit Injektionen verbundenen Nebenwirkungen. So sind zum Beispiel Blutungen an der Einstichstelle möglich, es muss eine Aspiration vorgesehen sein, um versehentliche intravasale Injektionen zu vermeiden und es können Krankheitserreger oder allgemein Keime über die Nadel übertragen werden. Insbesondere letzteres hat dazu geführt, dass häufig vorbeugend Antibiotika zur Vermeidung von Infektionen der Kastrationswunde gegeben werden.

Ferner ist zu berücksichtigen, dass die verwendeten Injektionsnadeln bereits nach wenigen Anwendungen erheblich abstumpfen und dadurch beim Tier unnötiger Weise stärkere Schmerzen beim Einstich verursachen.

Die vorliegende Erfindung hat daher, durch Bereitstellen eines besonders angepassten Kit-of-Parts, eine Verbesserung dieser Situation, sowohl hinsichtlich des Tierwohls, als auch hinsichtlich der Wirtschaftlichkeit zum Ziel.

Dieses Ziel wird durch das Kit-of-Parts gemäß dem beigefügten Anspruch 1 verwirklicht. Vorteilhafte Ausgestaltungen sind Gegenstand der auf diesen rückbezogenen Ansprüche sowie der nachfolgenden, ausführlichen Beschreibung der Erfindung.

Bei dem erfindungsgemäßen Kit-of-Parts hat man sich insbesondere die Tatsache zu Nutze gemacht, dass die Haut von unter 8 Tage alten Ferkeln im Bereich des Skrotums extrem dünn ist und eine transdermale Injektion eines Wirkstoffs letzteren vorteilhafter Weise in den subkutanen Bereich des Bindegewebes weit unter der Haut befördert. Dies erlaubt eine wirkungsvolle Lokalanästhesie, da eine räumlich ausreichend ausgedehnte Verteilung des Lokalanästhetikums erfolgt. Hierdurch ergibt sich überraschender Weise auch eine sehr hohe und vor allem im Vergleich zu einer Vollnarkose länger anhaltende Schmerzreduktion.

Die Injektion des Lokalanästhetikums erfolgt dorsal der Hoden, genauer in leicht medio-dorsaler Richtung, beiderseits der Raphe scroti. Bereits mit einem geringen Injektionsvolumen von 0,2 ml wird durch das Einbringen des Lokalanästhetikums mittels Luftdruck eine hervorragende Verteilung im Untergewebe erreicht. Im Vergleich dazu setzt eine Injektion per Nadel eine depotartige Quaddel, wodurch, bei gleicher Wirkstoffkonzentration, weit größere Volumina zum Erreichen eines ähnlichen Anästhetischen Effekts erforderlich sind.

Das erfindungsgemäße Kit-of-Parts ermöglicht somit bereits eine besondere Wirtschaftlichkeit, da ein schnellerer Wirkungseintritt bei geringerer Dosierung zu verzeichnen ist.

Ziel der Lokalanästhesie ist die örtliche Betäubung und die möglichst vollständige Schmerzreduktion während des chirurgischen Eingriffs der Kastration. Genauer bedeutet das die lokale Betäubung der Skrotalhaut, des Unterhautgewebes, des Processus vaginalis sowie des Samenstrangs mit Muskulatur.

Damit dieses zuverlässig erreicht wird müssen für die möglichst vollständige Schmerzreduktion die Nervenbahnen, die die obigen Strukturen versorgen, lokal betäubt werden.

Hier sind insbesondere der Nervus pudendus, der hauptsächlich die Skrotalhaut inerviert, der Nervus genitofemoralis und insbesondere dessen Ramus genitalis, welcher den Processus vaginalis, den Samenstrang, die Hoden und ebenfalls die Skrotalhaut inerviert, zu nennen.

Überraschenderweise hat sich gezeigt, dass diese im Bindegewebsbereich zwischen Haut und Oberschenkelmuskulatur liegenden Nerven mittels nadelloser, transdermaler Injektion mit kleinen Wirkstoffvolumina und -mengen optimal erreicht werden und so eine Schmerzreduktion bei der Kastration sicher, wirkungsvoll und auch wirtschaftlich erreicht wird.

Dabei sorgt eine breite Verteilung des Lokalanästhetikums im Gewebe für eine rasche und und optimale anästhetisierende Wirkung. Hieraus folgt ferner, dass die Hoden während des Eingriffs nicht krampfhaft zurückgezogen werden und der Samenstrang und die umgebende Muskulatur, insbesondere der Musculus cremaster, eine deutliche Erschlaffung zeigen. Dieses erleichtert wiederum den nachfolgenden chirurgischen Eingriff in vorteilhafter Weise.

Das erfindungsgemäße Kit-of-Parts umfasst eine Vorrichtung für nadellose Injektionen in schneller Abfolge mit einer Einrichtung zum Identifizieren des mittels der Vorrichtung zu verabreichenden Wirkstoffs sowie einer Auslöseeinrichtung zum Auslösen einer Injektion und ein Behältnis mit einem Lokalanästhetikum, das mit der Vorrichtung verbindbar ist und eine Einrichtung zum Identifizieren des Lokalanästhetikums und der Charge desselben aufweist.

Das Behältnis ist bevorzugt eine zur direkten Anwendung mit dem Lokalanästhetikum befüllte und konfektionierte Ampulle, insbesondere eine mit einem Septum versehene Durchstechampulle, die direkt in die Vorrichtung einsetzbar ist. In einer bevorzugten Weiterbildung ist die Vorrichtung so ausgestaltet, dass selbige handelsübliche Medikamentenampullen verschiedener Größen, wie beispielsweise 10, 20, 50 oder 100 ml, aufnehmen kann.

Hierdurch ergibt sich für den Anwender eine hinreichende Flexibilität in der Anwendung und es kann beispielsweise eine Darreichungsgröße gewählt werden, die der zu erwartenden Anzahl der aufeinander folgenden Anwendungen entspricht.

In einer Ausgestaltung der Vorrichtung für nadellose Injektionen in schneller Abfolge erfolgt das Auslösen der Injektion durch Betätigen einer Auslöseeinrichtung, beispielsweise eines Abzugs oder eines Auslöseknopfs auf mechanische oder elektronische Weise.

In einer unabhängigen, besonderen Ausgestaltung der Vorrichtung ist die Auslöseeinrichtung zum Auslösen der Injektion eine solche, die auf Gegendruck auslöst. Das heißt, das Auslösen erfolgt durch Aufdrücken der Vorrichtung auf die vorgesehene Injektionsstelle und nicht durch Betätigen einer Abzugseinrichtung oder dergleichen, wodurch sich die Handhabung der Vorrichtung erleichtert und ein Fehl- oder Falschauslösen praktisch unmöglich gemacht wird.

Um die Sicherheit bei der Injektion sowohl für den Anwender als auch für das Tier weiter zu erhöhen ist in einer weiteren, unabhängigen Ausgestaltung der Erfindung der Auslösemechanismus so ausgestaltet, dass erst bei vorliegen eines zuvor festgelegten Gegendrucks ein Betätigen der Auslöseeinrichtung erfolgen kann. Hiermit wird zum einen sichergestellt, dass die Vorrichtung korrekt für eine Injektion plaziert ist, und zum anderen wird ein irrtümliches Auslösen einer Injektion durch diese doppelte Sicherung so gut wie ausgeschlossen.

Nachfolgend werden weitere, optionale Merkmale und Ausgestaltungen der Vorrichtung nadellose Injektionen in schneller Abfolge beschrieben, die einzeln und unabhängig, aber auch in beliebiger Kombination, mit den oben bereits beschriebenen Merkmalen der Vorrichtung verwirklicht sein können. In diesem Zusammenhang ist festzustellen, dass in Abhängigkeit von der jeweils gewählten Kombination besondere Vorteile erhalten werden. Die Vorrichtung ist daher bevorzugt modular aufgebaut, so dass diese an die individuellen Bedürfnisse des Anwenders anpassbar ist.

Besonders bevorzugt ist, wenn die erfindungsgemäß in dem Kit-of-Parts enthaltene Vorrichtung eine Einstelleinrichtung zum Verändern des Drucks, mit dem das Lokalanästhetikum verabreicht wird, aufweist, weil hierdurch eine besonders einfache Handhabung möglich ist. Beispielsweise können Ferkel zur Zeit des vorgesehenen Eingriffs normalgewichtig oder leicht untergewichtig sein, wodurch sich aufgrund der daraus resultierenden Gewebeunterschiede bei festgelegtem Applikationsdruck unterschiedliche Eindringtiefen und Verteilungen im Injektionsbereich ergeben können. Eine, vorzugsweise zweistufige, Veränderbarkeit des Applikationsdrucks, mit dem das Lokalanästhetikum beaufschlagt wird, ist daher für eine optimale Lokalanästhesie unter unterschiedlichen Bedingungen zumindest förderlich. Dabei ist der Applikationsdruck in der werksseitigen Normaleinstellung die in der Regel verwendete Einstellung bei normalgewichtigen Ferkeln, während der Applikationsdruck für untergewichtige Ferkel zum Beispiel um etwa 5, 10 oder 20 % verminderbar ist.

Der Applikationsdruck ist Geräteabhängig und hängt beispielsweise von der Geometrie, insbesondere dem Durchmesser der Austrittsdüse ab und kann daher nicht absolut angegeben werden.

Es hat sich als von Vorteil erwiesen, wenn die eingangs allgemein beschriebene Vorrichtung in einer Weiterbildung eine Einrichtung zum Festlegen der zu verabreichenden Dosis des Lokalanästhetikums, oder allgemein des Wirkstoffs, aufweist. Obwohl es durchaus möglich ist mit einer werksseitig fest eingestellten Dosismenge zu arbeiten, erlaubt eine einstellbare Dosiereinrichtung eine besonders leichte Anpassung des Injektionsvolumen an den jeweilig verwendeten, speziellen Wirkstoff oder auch an unterschiedliche Konzentrationen des Wirkstoffs, das heisst des Lokalanästhetikums.

Eine Zähleinrichtung zum Erfassen der verabreichten Wirkstoffmenge und/oder der Anzahl der abgegebenen Injektionen kann ebenfalls vorgesehen sein und ist zum Erfassen des eingesetzten Wirkstoffs, das heißt zum Beispiel auch für die genaue Dokumentation, sinnvoll und von Vorteil.

In einer anderen unabhängigen Ausgestaltung umfasst die in der Vorrichtung enthaltene Einrichtung zum Identifizieren einer Wirkstoffcharge eine RFID-Leseeinrichtung. Hiermit wird die eindeutige Identifizierung des zu verabreichenden Wirkstoffs und somit dessen Lückenlose Dokumentation sichergestellt, wenn das Behältnis für das Lokalanästhetikum einen entsprechend ausgebildeten RFID-Chip umfasst. Dieser RFID-Chip ist bevorzugt im Inneren des Behälters mit dem Lokalanästhetikums angeordnet, um einer Manipulation vorzubeugen oder praktisch unmöglich zu machen.

Obwohl die Einrichtung zum Identifizieren einer Wirkstoffcharge in jeder dem Fachmann geläufigen Form ausgestaltet sein kann, z.B. mittels optischer Sensoren, CCD-Chips und dergleichen, ist eine RFID-Leseeinrichtung hierfür aber allgemein bevorzugt, da RFID-Tags günstig und in kleiner Größe erhältlich sind.

Mit dem erfindungsgemäßen Kit-of-Parts wird dessen Verwendung in einem Verfahren zum Kastrieren von Jungebern ermöglicht, weil eine lückenlose Dokumentation der abgegebenen und/oder eingesetzten Lokalanästhetika hinsichtlich Menge und Herkunft sichergestellt ist und zum Beispiel besonders geschulte Fachleute, wie Landwirte, mit der Durchführung der Kastration, einschließlich der lokalen Betäubung, ohne Hinzuziehen eines Tierarztes betraut werden können. Dies erhöht die Wirtschaftlichkeit im Ferkelerzeugerbetrieb.

Aus praktischen Gründen ist es ferner vorteilhaft, wenn die Vorrichtung eine Speichereinrichtung zum Aufzeichnen der Anzahl der durchgeführten Injektionen, der verabreichten Wirkstoffmenge und/oder der Wirkstoffcharge aufweist, wodurch ein lückenloses Erfassen und Dokumentieren sämtlicher mit der Abgabe des Lokalanästhetikums anfallender Daten, einschliesslich der Bedienerdaten, erlaubt.

In einer weiteren, unabhängigen Ausgestaltung der vorliegenden Erfindung ist die RFID-Leseeinrichtung sowie die Speichereinrichtung derart angepasst, dass auch die RFID-Tags der Ferkelmarkierungen mit der erfindungsgemäßen Vorrichtung gelesen und verarbeitet werden können. Diese Ausgestaltung bietet den besonderen Vorteil, dass eine genaue Zuordnung von Ferkel und verabreichter Dosis des Lokalanästhetikums erfolgt und Dokumentiert wird, so dass eine umfassende Dokumentation auf einfache Art ermöglicht wird und sich sehr effizient in einen betrieblichen Ablauf integrieren lässt.

Auch eine Schnittstelle zum Auslesen von erfassten Daten ist Vorteilhaft, um die erfassten Daten beispielsweise über drahtlose Verbindung oder mittels Kabel an eine Externe Einrichtung zum Erfassen, Verarbeiten und Auswerten der Daten zu übergeben.

Die in dem erfindungsgemäßen Kit-of-Parts enthaltene Vorrichtung für nadellose Injektionen in schneller Abfolge wird bevorzugt elektrisch betrieben, wobei es bevorzugt ist, wenn die Vorrichtung einen austauschbaren Akku als Energiequelle nutzt. Hierdurch wird eine hohe Anwendungsflexibilität sichergestellt.

Das in dem Kit-of-Parts enthalten Behältnis mit einem Lokalanästhetikum ist bevorzugt mit einem oder mehreren Lokalanästhetika, ausgewählt aus der Gruppe, bestehend aus Lidocain, Mepivacain, Prilocain, Articain, Bupivacain, Ropivacain, Etidocain, Dyclonin, Procain, Benzocain, 2-Chlorprocain, Oxybuprocain, Tetracain und Fomocain befüllt. Besonders bevorzugt sind Procain und/oder Lidocain.

Dabei ist es bevorzugt, wenn dem Lokalanästhetikum, welches auch eine beliebige Mischung aus zwei oder mehr der oben genannten Verbindungen sein kann, ein Sperrkörper beigefügt ist, weil hierdurch die ohnehin schon geringe Blutungsneigung bei der nadellosen Injektion noch weiter herabgesetzt wird, so dass an der Injektionsstelle im allgemeinen praktisch keine Blutung mehr festzustellen ist.

Wenn das in dem Behältnis enthaltene Lokalanästhetikum einen Sperrkörper enthält, so ist dieses ausgewählt aus der Gruppe, bestehend aus Epinephrin, Noradrenalin und Adrenalin, wobei Epinephrin bevorzugt ist. Der besondere Vorteil der der Gegenwart eines Sperrkörpers liegt in ihrer vasokontraktiven Wirkung, die der vasodilatorischen Wirkung der Lokalanesthetika entgegenwirkt und so eine mögliche Blutungsneigung noch weiter verringert.

Es ist auch bevorzugt die genannten Lokalanästhetika in ihrer pharmazeutisch üblichen Form einzusetzen, zum Beispiel in Form ihrer pharmazeutisch verträglichen Additionssalze, insbesondere in der Form ihrer Hydrochloride.

Die Lokalanästhetika werden dabei üblicherweise in Konzentrationen von 20 bis 40 mg/ml verwendet, bevorzugt in einer Konzentration von 20 mg/ml und sofern ein Sperrkörper zum Einsatz kommt, ist dieser in einer Konzentration von 0,015 bis 0,050 mg/ml enthalten, bevorzugt in einer Konzentration von 0,025 mg/ml.

Zusammenfassend ist somit festzustellen, dass das erfindungsgemäße Kit-of-Parts erlaubt, ein Kastrieren von Jungebern unter Verwendung einer Vorrichtung für nadellose Injektionen in schneller Abfolge besonders tierschonend und wirtschaftlich durchzuführen, da die Lokalanästhesie nur gering invasiv ist und nur eine kaum sichtbare Injektionsstelle bildet, welche sich gewöhnlich nur durch eine leichte Rötung zeigt, wenn überhaupt. Dabei treten Infektionen an der Applikationsstelle extrem selten auf, weil z.B. keine Keime übertragen werden können, wie es bei einer Injektion mittels Nadel häufiger vorkommt.

Das Verabreichen des Lokalanästhetikum ist auch nur minimal traumatisch, was sich durch das Ausbleiben einer Schmerzreaktion während der Injektion zeigt.

Es gibt keine oder nur äusserst geringe Blutungen an der Applikationsstelle des Lokalanästhetikums und es ist ein hoher Grad an Schmerzfreiheit sichergestellt, und das trotz geringer Dosierung. Diese vorteilhafte Wirkung beruht auf der hohen Gewebeverteilung des Lokalanästhetikums, wie sie nur mittels nadelloser Injektion erzielbar ist.

Ferner lässt sich die Menge des zu verabreichenden Lokalanästhetikums exakt steuern und gegebenenfalls individuell einstellen, wobei die Verwendung sehr sicher Fehlinjektionen vermeidet, wenn die Injektion durch ein Gegendruck-Systemausgelöst wird.

Durch die breite Gewebeverteilung des Lokalanästhetikums und dem konstanten Druck, mit dem die Injektion erfolgt, ist auch die Gefahr einer ungenauen Situierung der Injektionsstelle vorteilhafter Weise minimiert, da das Lokalanästhetikum immer gleich tief eindringt. Diese Genauigkeit in der Gewebeverteilung bei der transdermalen Injektion des Lokalanästhetikums wird weiter durch die Einstellbarkeit des Appliaktionsdrucks verbessert, die wie bereits beschrieben auch bei untergewichtigen Ferkeln eine optimale Verteilung des Lokalanästhetikums sicherstellt.

Das erfindungsgemäße Kit-of-Parts erlaubt darüber hinaus auch einen sehr effizienten Einsatz, weil nur eine Person für die Ausführung erforderlich ist und die Lokalanästhesie maximal 10 Sekunden pro Tier beansprucht. Nach erfolgter Injektion werden die Tiere dann bis zur Entfaltung der vollen betäubenden Wirkung, im allgemeinen etwa 10 Minuten, in einer Ruhebox abgesetzt. Anschließend kann die Kastration dann wie üblich vorgenommen werden.

Wegen der geringen erforderlichen Mengen an Arzneimittel, der hohen Arbeitsgeschwindigkeit und der einfachen Durchführung, die durch das erfindungsgemäße Kit-of Parts erreicht werden, ist der Einsatz in der im Ferkelerzeugerbetrieb auch sehr wirtschaftlich.

Insgesamt erlaubt die in dem Kit-of-Parts enthaltene Vorrichtung nadellose Injektionen in schneller Abfolge in Kombination mit dem ein Lokalanästhetikum enhaltenden Behältnis eine besonders hohen Transparenz und sehr gute Nachvollziehbarkeit hinsichtlich des eingesetzten Lokalanästhetikums, insbesondere, wenn die Vorrichtung über entsprechende, vorzugsweise elektronische, Zähl-, Speicher- und/oder Übermittlungseinrichtungen aufweist, so dass der Verbrauch je nach Ausgestaltung der Auswertung Anwenderspezifisch und/oder Bediener- oder Betriebsspezifisch lückenlos dokumentierbar ist.

Schließlich ist die Verwendung des erfindungsgemäßen Kit-of-Parts in einem Verfahren zum Kastrieren von Jungebern problemlos und mit nur äußerst geringen Mehraufwand gegenüber der betäubungsmittelfreien Kastration durchführbar, bereitet keine Akzeptanzprobleme von Seiten des Verbrauchers und fördert das Tierwohl.

## Patentansprüche

1. Kit-of-Parts, umfassend
eine Vorrichtung für nadellose Injektionen in schneller Abfolge mit einer Einrichtung zum Identifizieren des mittels der Vorrichtung zu verabreichenden Wirkstoffs sowie einer Auslöseeinrichtung zum Auslösen einer Injektion und
ein Behältnis mit einem Lokalanästhetikum, das mit der Vorrichtung verbindbar ist und eine Einrichtung zum Identifizieren des Lokalanästhetikums und der Charge desselben aufweist, wobei die Vorrichtung ferner
eine Zähleinrichtung zum Erfassen der verabreichten Wirkstoffmenge und/oder der Anzahl der abgegebenen Injektionen
eine Speichereinrichtung zum Aufzeichnen der Anzahl der durchgeführten Injektionen, der verabreichten Wirkstoffmenge, der Wirkstoffcharge und/oder des Bedieners der Vorrichtung und eine Schnittstelle zum Auslesen von erfassten Daten
aufweist.

2. Kit-of-Parts nach Anspruch 1, wobei die Auslöseeinrichtung ein Auslösen der Injektion mittels einer Gegendruckeinrichtung bewirkt.

3. Kit-of-Parts nach Anspruch 1 oder 2, wobei die Vorrichtung für nadellose Injektionen in schneller Abfolge ferner, jeweils unabhängig, ein oder mehrere der folgenden Merkmale aufweist:
(a) eine Einstelleinrichtung zum Verändern des Drucks, mit dem das Lokalanästhetikum verabreicht wird
(b) eine Einstelleinrichtung zum Festlegen der zu verabreichenden Dosis des Lokalanästhetikums.

4. Kit-of-Parts nach einem der vorhergehenden Ansprüche, wobei die Einrichtung zum Identifizieren des Lokalanästhetikums und der Charge innenliegend in dem Behälter angeordnet ist.

5. Kit-of-Parts nach einem der vorhergehenden Ansprüche, wobei die Einrichtung zum Identifizieren des Lokalanästhetikums und der Charge von einem RFID-Chip gebildet ist.

6. Kit-of-Parts nach einem der vorhergehenden Ansprüche, wobei das Behältnis neben dem Lokalanästhetikum einen Sperrkörper enthält.

7. Kit-of-Parts nach einem der vorhergehenden Ansprüche, wobei das Lokalanästhetikum ein oder mehrere Lokalanästhetika ausgewählt aus der Gruppe, bestehend aus Lidocain, Mepivacain, Prilocain, Articain, Bupivacain, Ropivacain, Etidocain, Dyclonin, Procain, Benzocain, 2-Chlorprocain, Oxybuprocain, Tetracain und Fomocain, ist.

8. Kit-of-Parts nach einem der vorhergehenden Ansprüche, wobei der Sperrkörper von eine oder mehrere Verbindungen, ausgewählt aus der Gruppe, bestehend aus Epinephrin, Noradrenalin und Adrenalin, umfasst.

9. Kit-of-Parts nach einem der vorhergehenden Ansprüche, wobei das ein Lokalanästhetikum enthaltende Behältnis ein Volumen von 10, 25, 50 oder 100 ml aufweist.

10. Kit-of-Parts nach einem der vorhergehenden Ansprüche, wobei das Lokalanästhetikum in dem Behälter in einer Konzentration von 20,0 mg/ml und, sofern vorhanden, der Sperrkörper in eine Konzentration von 0,025 mg/ml.

11. Verwendung eines Kit-of-Parts nach einem der vorhergehenden Ansprüche in einem Verfahren zum Kastrieren von Ferkeln in einem Alter von unter 8 Tagen.
